# EUROPEAN PATENT SPECIFICATION

(11) **EP 3 586 761 B1**
(45) Date of publication and mention of the grant of the patent: **30.07.2025**
(21) Application number: 19183055.3
(22) Date of filing: 27.06.2019
(51) Int. Cl.: A61B 10/02, A61B 90/11, A61B 90/17, A61B 6/00, A61B 6/04, A61B 8/08, A61B 34/20, A61B 8/00, G16H 40/63, A61B 17/34

(54) **SYSTEM AND METHOD FOR SELECTING A PATIENT POSITION AND AN EQUIPMENT CONFIGURATION FOR A MEDICAL PROCEDURE**
SYSTEM UND VERFAHREN ZUR AUSWAHL EINER PATIENTENPOSITION UND EINER AUSRÜSTUNGSKONFIGURATION FÜR EINEN MEDIZINISCHEN EINGRIFF
SYSTÈME ET PROCÉDÉ DE SÉLECTION DE POSITION D'UN PATIENT ET CONFIGURATION D'ÉQUIPEMENT POUR PROCÉDURE MÉDICALE

(30) Priority: 28.06.2018 US 201816021904
(43) Date of publication of application: 01.01.2020
(73) Proprietor: GE Precision Healthcare LLC, Waukesha, Wisconsin 53188-1615 (US)
(72) Inventor: VANCAMBERG, Laurence, 78530 Buc (FR); SANTI, Karima, 78530 Buc (FR)
(74) Representative: Fennell, Gareth Charles

(56) References cited:
- US-A1- 2003 195 644
- US-A1- 2010 329 419
- US-A1- 2012 014 499
- US-A1- 2014 098 932
- US-A1- 2016 166 222
- US-A1- 2017 172 530

## Description

### BACKGROUND

### TECHNICAL FIELD

Embodiments of the invention relate generally to the medical diagnostic equipment, and more specifically, to systems and methods for selecting a patient position and an equipment configuration for a medical procedure.

### DISCUSSION OF ART

In the medical field there are often numerous pieces of mechanized diagnostic equipment deployed for use in patient care. Often, multiple pieces of equipment made by different manufacturers are employed by practitioners during the examination of a patient. In a typical arrangement, patient positioning equipment is coupled with the usage of diagnostic equipment, e.g., a mammogram device, dental x-ray machine, endoscopy, ultrasound guided biopsy practice, etc.

For example, the process of obtaining high quality mammographic images or biopsies from breast tissue generally requires a practitioner to orchestrate multiple pieces of equipment while also ensuring proper patient positioning, and equipment configuration. In particular, a practitioner may coordinate movements of a mammographic gantry, including lift, rotation, and angle. Further controls are required to control breast compression, imaging parameters (i.e., field of view, lighting, dosage); and in the case of ultrasound-guided biopsies, biopsy probe movement, suction controls, and ultrasonic imaging parameters. Patients are often positioned and supported during interventional mammographic procedures using mechanized chairs or tables.

In the case of a breast biopsy, a mammographic practitioner must account for the location of the lesion, the type of equipment available in the room, and the constraints upon such equipment in order to determine an optimal position for a patient to hold, as well as the correct configuration for the biopsy equipment used during a biopsy procedure.

Traditionally, a medical assistant must prepare the room prior to the **patient's arrival. The assistant, however,** must often wait for the patient to arrive at the room containing the medical equip**ment before knowing the patient's positional** preferences, e.g., sitting or recumbent, for a particular procedure, e.g., a mammogram and/or biopsy procedure. Moreover, the medical assistant may not know the particular equipment configuration required, or preferred by the controlling entity of the medical equipment (e.g., the preferences of hospital staff), for the medical procedure to be performed, e.g., a vertical or horizontal approach for a biopsy needle. Further, the **patient's position and/or the equip**ment configuration may be determined by the **patient's morphology and/or lesion location, which may be unknown to the medical** assistant.

Thus, medical assistants must often guess **both the patient's desired** position and/or the correct equipment configuration prior to the arrival of the patient and/or the preferences of the technologist/operator performing the medical procedure. As will be understood, however, incorrect guesses/assumptions by the medical assistant may require the medical equipment to be repositioned upon arrival of the patient and/or operator preforming the medical procedure, which in turn, generally decreases the efficiency of the medical procedure, e.g., increases the amount of time the patient, medical assistant, and/or operator performing the medical procedure, must spend in the room. In some instances, incorrect positioning of the patient and/or configuration of the medical equipment may result in a defective procedure, e.g., a biopsy needle may not reach its intended target, thus requiring the procedure to be re-started/redone. While many experienced medical assistants may be able to guess the correct patient position and/or equipment configuration for a particular procedure with a high rate of success, many inexperienced medical assistants will usually guess incorrectly. US 2016/166222 A1 describes an X-ray imaging apparatus which guides the patient posture using a visual and/or audible interaction device during execution of an X-ray imaging procedure such as mammography, and provides the patient with information about the procedure during the procedure's execution, thereby improving the patient experience while reducing the workload of an operator. The X-ray imaging apparatus may include an X-ray tube, an X-ray detector, and an arm including an upper end within which the X-ray tube is disposed, and a lower end within which the X-ray detector is disposed. An illumination unit may be configured to provide an indication of a movement direction of the arm, responsive to a user input for moving the arm in the movement direction. A controller may control the illumination unit.

What is needed, therefore, is an improved system and method for selecting a patient position and an equipment configuration for a medical procedure.

### BRIEF SUMMARY OF THE INVENTION

In an embodiment, a system for selecting a patient position and an equipment configuration for a medical procedure is provided. The system includes a controller, and a memory device operative to store an application. The application adapts the controller to retrieve, from the memory device, one or more possible patient positions for the medical procedure and one or more possible equipment configurations for the medical procedure. The application further adapts the controller to select, based at least in part on one or more parameters of the medical procedure, a patient position from the possible patient positions and an equipment configuration from the possible equipment configurations; and to generate an indicator that conveys the selected patient position and the equipment configuration. In the presently claimed invention, the medical procedure is a breast biopsy.

In another embodiment, a method for selecting a patient position and an equipment configuration for a medical procedure is provided. The method includes retrieving, via a controller from a memory device, one or more possible patient positions for the medical procedure and one or more possible equipment configurations for the medical procedure. The method further includes selecting, via the controller and based at least in part on one or more parameters of the medical procedure, a patient position from the possible patient positions and an equipment configuration from the possible equipment configurations. The method further includes generating, via the controller, an indicator that conveys the selected patient position and the equipment configuration. In the presently claimed invention, the medical procedure is a breast biopsy.

Also disclosed herein, but not forming part of the claimed invention, is a non-transitory computer readable medium storing instructions. The stored instructions adopt a controller to retrieve, from a memory device, one or more possible patient positions for a medical procedure and one or more possible equipment configurations for the medical procedure. The stored instructions further adapt the controller to select, based at least in part on one or more parameters of the medical procedure, a patient configuration from the possible patient positions and an equipment configuration from the possible equipment configurations, and to generate an indicator that conveys the selected patient position and equipment configuration.

### DRAWINGS

The present invention will be better understood from reading the following description of non-limiting embodiments, with reference to the attached drawings, wherein below:
FIG. 1 is a perspective view of a system for selecting a patient position and an equipment configuration for a medical procedure, in accordance with an embodiment of the presently claimed invention;
FIG. 2 is a diagram of medical equipment used to perform the medical procedure of FIG. 1;
FIG. 3 is a flow chart depicting a method of selecting a patient position and an equipment configuration for a medical procedure utilizing the system of FIG. 1, in accordance with an embodiment of the present invention;
FIG. 4 is a diagram of a biopsy probe of the medical equipment of FIG. 2, wherein the biopsy probe has one or more possible configurations, in accordance with an embodiment of the present invention;
FIG. 5 is a diagram of an embodiment of the biopsy probe of FIG. 4, wherein the biopsy probe is a needle, in accordance with an embodiment of the present invention; and
FIG. 6 is a diagram of another embodiment of the biopsy probe of FIG. 4, wherein the biopsy probe is a hook, in accordance with an embodiment of the present invention.

### DETAILED DESCRIPTION

Reference will be made below in detail to exemplary embodiments of the invention, examples of which are illustrated in the accompanying drawings. Wherever possible, the same reference characters used throughout the drawings refer to the same or like parts, without duplicative description.

**As used herein, the terms "substantially," "generally," and "about"** indicate conditions within reasonably achievable manufacturing and assembly tolerances, relative to ideal desired conditions suitable for achieving the functional purpose of a component or **assembly. As used herein, "electrically coupled", "electrically connected", and "electrical communication"** mean that the referenced elements are directly or indirectly connected such that an electrical current may flow from one to the other. The connection may include a direct conductive connection, i.e., without an intervening capacitive, inductive or active element, an inductive connection, a capacitive connection, and/or any other suitable electrical connection. Intervening components may be present. **Additionally, the term "medical procedure", as used** herein, refers to a series of steps involving a patient and equipment that is intended to achieve a desired medical outcome. Examples of medical procedures include mammograms, breast biopsies, stent deployments, dental x-**rays, etc. The term "patient position", as used herein, refers to the physical** configuration/position of a patient during a medical procedure. The terms **"medical equipment" and/or "equipment", as** used herein, refers to any type of device used to perform a medical procedure.

Further, while the embodiments disclosed herein are described with respect to a breast biopsy system and procedure, it is to be understood that embodiments not forming part of the present invention may be applicable to other types of medical procedures which involve the selection/pairing of a patient position and medical equipment configuration. Further still, as will be appreciated, the related imaging systems may be used to analyze tissue generally and are not limited to human tissue.

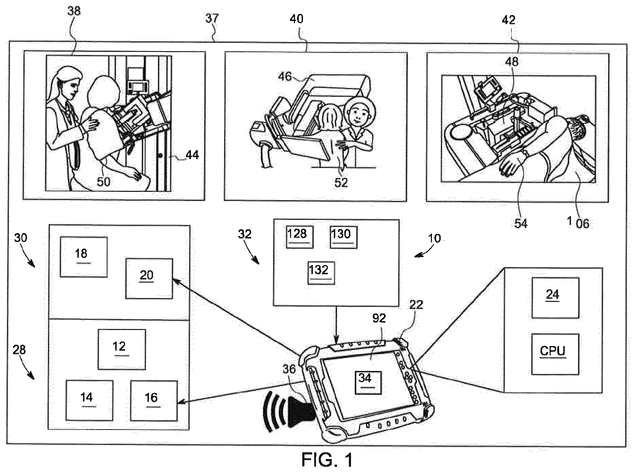

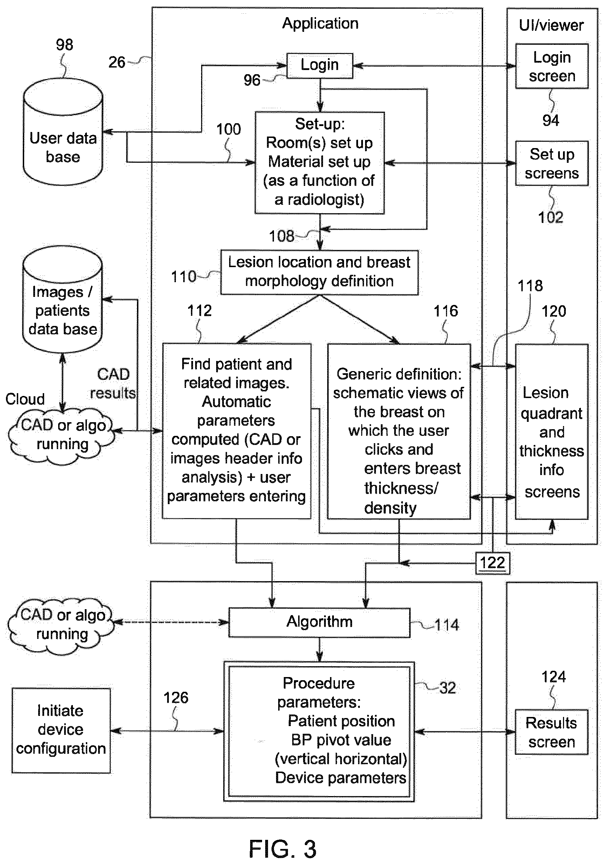

Referring now to FIG. 1, the major components of a system 10 for selecting a patient position 12, 14, 16 and an equipment configuration 18, 20 for a medical procedure, e.g., a mammography exam, breast biopsy, etc., are shown. The system 10 includes a controller 22 and a memory device 24. The memory device 24 stores an application 26 (FIG. 3) that adapts the controller 22 to retrieve, from the memory device 24, one or more possible patient positions 28, and one or more possible equipment configurations 30 for the medical procedure. As will be understood, equipment configurations 30 may include, but are not limited to: equipment orientations, e.g., a vertical, horizontal, or other directional approach/trajectory for a biopsy probe to traverse in order to reach a target/lesion site; probe size, e.g., a needle gauge and/or length; an amount of anesthesia and/or dye to be injected into the patient, and/or other appropriate configuration settings for medical equipment. The application 26 further adapts the controller 22 to select, based at least in part on one or more parameters 32 of the medical procedure, a patient position, e.g., patient position 12, from the possible patient positions 28, and an equipment configuration, e.g., equipment configuration 18, from the possible equipment configurations 30; and to generate an indicator 34, 36 that conveys the selected patient position 12 and the equipment configuration 18. In other words, in embodiments, the controller 22 generates an indictor 34, 36 that informs a medical assistant as to the patient position and equipment configuration to be used to perform/conduct a particular medical procedure for a particular patient. The indicator may be a visual indicator 34, e.g., message box, light, etc., and/or an audio indicator 36, e.g., musical chime, computerized voice, etc.

As shown in FIG. 1, the controller 22 may be a mobile electronic device, e.g., a tablet, smart phone, laptop, etc., which is used by an operator/technician/medical assistant to determine the most appropriate configuration for a medical procedure, i.e., how to best position a patient, how to configure the equipment used to accomplished a medical procedure on the patient, etc. For example, a radiologist assistant may work at a medical facility 37, **e.g., a hospital, doctor's office, etc., having a plurality of** examination/procedure rooms 38, 40, and 42. As will be appreciated, while FIG. 1 depicts the rooms 38, 40, and 42 within the same medical facility 37, it is to be understood that the rooms 38, 40, and 42 may be distributed across several facilities which may be operated by the same and/or different entities. Each of the rooms 38, 40, 42 may have the same and/or different medical equipment 44, 46, 48 that is operative/configured to perform a medical procedure on a patient 50, 52, 54.

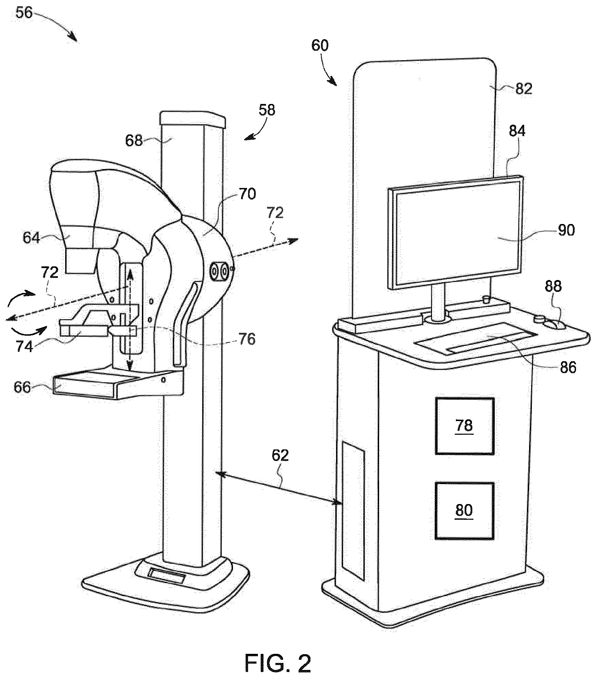

Turning to FIG. 2, an exemplary piece of medical equipment 56, e.g., a mammography imaging system/machine/device, in accordance with an embodiment of the present invention is shown. The mammography imaging system 56 may include an imaging device/system 58 and a controller 60. The imaging device 58 is operative to obtain one or more images of the patient 50, 52, 54 (FIG. 1), e.g., a human breast or other body part/area of interest. The controller 60 electronically communicates with the imaging device 58, via data link 62, which, in embodiments, may be a wired and/or wireless connection.

As further shown in FIG. 2, the imaging device 58 includes a radiation source/emitter 64 and a radiation detector 66. The radiation source 64 is operative to emit radiation rays and is selectively adjustable between one or more positions, e.g., the radiation source 64 may be mounted to a stand/support 68 via a rotatable mount 70 such that the radiation source 64 rotates about a longitudinal axis 72. The radiation detector 66 is operative to receive the radiation rays from the radiation source 64. In embodiments, the imaging device 58 may include one or more paddles 74 e.g., a compression plate, mounted to the stand 68 and slidably adjustable along axis 76 (and/or other axis/direction) so as to compress and/or restrain the patient 50, 52, 54 (FIG. 1), e.g., a breast, against the detector 66.

In embodiments, the controller 60 may be a workstation having at least one processor 78 and a memory device 80. In other embodiments, the controller 60 may be embedded / integrated into one or more of the various components of the imaging device 56 disclosed above. In embodiments, the controller 60 may be in electrical communication with the radiation source 64, radiation detector 66, the paddles 74, the controller 22 (FIG. 1) via link 62. In embodiments, the controller 60 may include a radiation shield 82 that protects an operator of the medical equipment 56 from the radiation rays emitted by the radiation source 64. The controller 60 may further include a display 84, a keyboard 86, mouse 88, and/or other appropriate user input devices, that facilitate control of the medical equipment 56 via a user interface 90.

Illustrated in FIG. 3 is a process flow chart depicting operation of the system 10, in accordance with an embodiment of the present invention. As shown, a practitioner, who may perform the medical procedure on the patient 50, 52, 54 (FIG. 1) may interact with a graphical user interface ("GUI") on controller apart from controller 22, e.g., a work station or other mobile device, designed to accept practitioner input, acknowledge the input, and then pass the input to controller 22 and/or 60, which may act upon the input and alter a user interface on controller 22 and/or 60. The user interface, for example, may be a GUI displayed on a monitor 92 (FIG 1) coupled to/disposed in controller 22, on display 84 (FIG. 2), or other electronic device capable of depicting an interactive GUI. In embodiments, the user interface may be hardware tailored to accept inputs specific to the method; for example, a physical switch which in a first state indicates that a patient preference is to sit upright (as shown in room 38 in FIG. 1), a second state indicates a patient preference to remain recumbent (as shown in room 42 in FIG. 1), and a third state indicates a patient preference to stand (as shown in room 40 in FIG. 1). As will be appreciated, it is contemplated that the user interface takes the form of a GUI displayed on the screen of a computer-driven display. Thus, areas of the GUI where a practitioner enters in commands or values as part of the method **are referred to via the generic term "screen",** although other forms of display, such as an LED readout, or indicator lights, may also be within the scope of practice.

As shown in FIG. 3, a practitioner may enter in login credentials at login screen 94 or may be authorized via biometric marker using a fingerprint scanner or facial or optical recognition apparatus (not shown). The credentials are passed to login protocol 96 which may retrieve user data from a local storage device (not shown) or from a user database 98 and which permits access to and initiates subsequent method steps. The login protocol 96 may also determine the credentials or a user category of the practitioner (e.g., technician, radiologist, doctor, nurse, etc.), thus setting the complexity and capability of the overall algorithm. For example, a technician may make initial decisions for configuration and provide inputs for variable values which are subsequently reviewed and approved by a physician or radiologist whereas actions dictated by a physician would be immediately accepted and acted upon. In embodiments, the medical assistant may enter in the name of the practitioner performing the medical procedure into the GUI, which in turn, may cause the GUI to **display the practitioner's** preferences for equipment configuration and/or patient position.

A series of examination room configurations is then retrieved 100 from storage either locally, from a remote memory storage unit (not shown), or from the same memory unit as user database 98. The possible configurations are displayed and selected in set up screens 102. By way of non-limiting example, an examination room 42 (FIG. 1) containing a combination of mammography imaging system 48 (FIG. 1) and patient support table 106 (FIG. 1) may be chosen as a room configuration. Room configuration data, such as mammography imaging system 48 type and tolerances, along with patient support table 106 movement capability, along with the presence and specifications of any accessory devices, is then stored as configuration data 108 and the practitioner proceeds into Lesion Location and Breast Morphology Definition protocol 110.

As part of Lesion Location and Breast Morphology Definition protocol 110, initial patient physiology data (e.g., images, computer-aided-detection data **("CAD"), header information, pre**-entered data, lesion location data, etc.) may be retrieved from local or offsite storage 112 and sent to algorithm 114 without further practitioner input. In embodiments, some, all, or none of the initial patient data may be overlaid over a generic model or representative image 116, e.g., a wireframe or other suitable depiction retrieved from a patient file in database 98, is then displayed 118 in the user interface as a screen or series of screens 120 configured to enable the practitioner to select and enter variable values (e.g. breast thickness, size, lesion location, distance to skin, etc.) to create final patient physiology data 122. Variables entered can include: target lesion quadrant, breast thickness, breast size, distance to skin, and other physiological or machine parameters necessary to the medical protocol performed. The patient physiology data 122 may or may not be combined with patient data retrieved from local or offsite storage 112 before being sent to algorithm 114.

In embodiments, patient data retrieved at 112 and patient data 122, singly or together, are combined with configuration data 108 via algorithm 114 to generate procedure parameters 32 (e.g., patient position and configuration, movements for patient support table 106, position settings for a gantry, biopsy probe pivot value (horizontal/vertical), hook vs. syringe type probe and size, etc.). As will be understood, however, one or more of the parameters 32 may be pre-determined and/or retrieved from an external database. The procedure parameters 32 are displayed via a results screen 124 which, upon approval by the practitioner, or supervisor, may initiate implementation 126 of the configuration conforming to the procedure parameters 32.

Accordingly, returning briefly back to FIG. 1, in embodiments, the parameters 32 for the medical procedure may include at least one of room configuration data 128, patient physiology data 130, medical device data 132, and/or other parameters that impact and/or control the medical procedure to be performed. Room configuration data 128 may include the location of the medical equipment 44, 46, 48, the size/shape of the room, 38, 40, 42, and other appropriate properties of the room 38, 40, 42 which may affect the medical procedure.

In embodiments, the one or more possible patient positions 28 may include at least one of sitting 12, recumbent 14, and/or other suitable/appropriate patient positions for the medical procedure, e.g., standing 16 or kneeling. In embodiments, the possible patient positions 28 may convey a direction the patient 50, 52, 54 is to face with respect to the medical equipment 44, 46, 48, e.g., to the left or right.

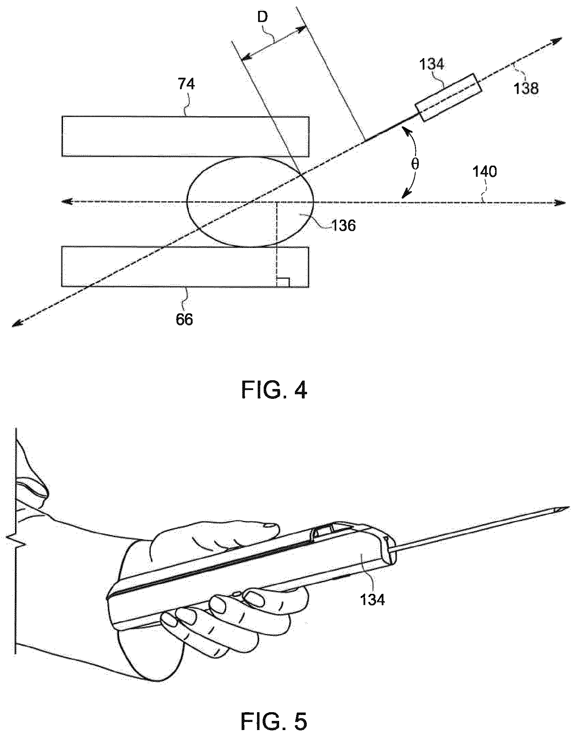

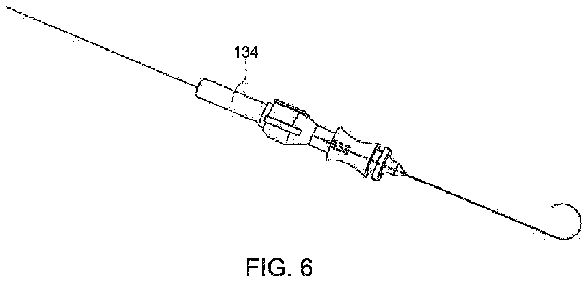

Moving to FIGS. 4-6, as stated above, in the presently claimed invention, the medical procedure is a breast biopsy. As will be understood, breast biopsy procedures may involve/utilize a biopsy probe 134 that penetrates/compresses a breast 136. Accordingly, in embodiments, the medical device data 132 (FIG. 1) may convey whether the biopsy probe 134 is a needle (as shown in FIG. 5), a hook (as shown in FIG. 6), e.g., a hook-wire for breast localization, and/or other appropriate type of biopsy probe. For example, in some embodiments, the medical device data 132 may convey the type, gauge, and/or length of the needle 134 (FIG. 5) to be used, e.g., 10g or a 7g. In embodiments, the medical device data 132 may be based on the options available in the particular room 38, 40, 42 (FIG. 1) and/or the preference of the patient 50, 52, 54 and/or the physician performing the medical procedure.

In embodiments, the possible equipment configurations 30 may include an approach angle θ (FIG. 4) for the biopsy probe 134, e.g., the angle at which a trajectory 138 of the biopsy probe 134 makes with a horizontal axis 140 of the breast 136. In embodiments, the approach angle θ may be substantially zero (0) degrees or substantially ninety (90) degrees. In other words, the selected equipment configuration for the biopsy probe 134 may be substantially vertical 18 (FIG. 1) or substantially horizontal 20 (FIG. 1). As will be understood, however, the approach angle θ may vary.

In embodiments, the patient physiology data 130 (FIG. 1) may include at least one of a lesion location, i.e., the target site of a biopsy, a breast 136 density, a breast 136 thickness, and an initial distance D (FIG. 4) between the biopsy probe 134 and the breast 136.

Finally, it is also to be understood that the system 10 may include the necessary electronics, software, memory, storage, databases, firmware, logic/state machines, microprocessors, communication links, displays or other visual or audio user interfaces, printing devices, and any other input/output interfaces to perform the functions described herein and/or to achieve the results described herein. For example, as previously mentioned, the system 10 may include at least one processor and system memory / data storage structures, which may include random access memory (RAM) and read-only memory (ROM). The at least one processor of the system 10 may include one or more conventional microprocessors and one or more supplementary co-processors such as math co-processors or the like. The data storage structures discussed herein may include an appropriate combination of magnetic, optical and/or semiconductor memory, and may include, for example, RAM, ROM, flash drive, an optical disc such as a compact disc and/or a hard disk or drive.

Additionally, a software application that adapts the controller to perform the methods disclosed herein may be read into a main memory of the at least one processor from a computer-readable medium. **The term "computer-readable medium",** as used herein, refers to any medium that provides or participates in providing instructions to the at least one processor of the system 10 (or any other processor of a device described herein) for execution. Such a medium may take many forms, including but not limited to, non-volatile media and volatile media. Non-volatile media include, for example, optical, magnetic, or opto-magnetic disks, such as memory. Volatile media include dynamic random access memory (DRAM), which typically constitutes the main memory. Common forms of computer-readable media include, for example, a floppy disk, a flexible disk, hard disk, magnetic tape, any other magnetic medium, a CD-ROM, DVD, any other optical medium, a RAM, a PROM, an EPROM or EEPROM (electronically erasable programmable read-only memory), a FLASH-EEPROM, any other memory chip or cartridge, or any other medium from which a computer can read.

While in embodiments, the execution of sequences of instructions in the software application causes at least one processor to perform the methods/processes described herein, hard-wired circuitry may be used in place of, or in combination with, software instructions for implementation of the methods/processes of the present invention. Therefore, embodiments of the present invention are not limited to any specific combination of hardware and/or software.

It is further to be understood that the above description is intended to be illustrative, and not restrictive. For example, the above-described embodiments (and/or aspects thereof) may be used in combination with each other. Additionally, many modifications may be made to adapt a particular situation or material to the teachings of the invention without departing from its scope.

For example, in an embodiment, a system for selecting a patient position and an equipment configuration for a medical procedure is provided. The system includes a controller, and a memory device operative to store an application. The application adapts the controller to retrieve, from the memory device, one or more possible patient positions for the medical procedure and one or more possible equipment configurations for the medical procedure. The application further adapts the controller to select, based at least in part on one or more parameters of the medical procedure, a patient position from the possible patient positions and an equipment configuration from the possible equipment configurations; and to generate an indicator that conveys the selected patient position and the equipment configuration. In certain embodiments, the one or more parameters of the medical procedure include at least one of: room configuration data; patient physiology data; and medical device data. In the presently claimed invention, the medical procedure is a breast biopsy. In certain embodiments, the one or more possible patient positions include at least one of sitting and recumbent. In certain embodiments, the possible equipment configurations include an approach angle for a biopsy probe and/or a needle length of the biopsy probe. In certain embodiments, the one or more parameters of the medical procedure include medical device data, and the application further adapts the controller to receive the medical device data via a user input device. In certain embodiments, the medical device data conveys whether the medical procedure utilizes a hook or a needle. In certain embodiments, the patient physiology data includes at least one of: a lesion location; a breast density; a breast thickness; and an initial distance between a biopsy probe and a breast. In certain embodiments, the controller is disposed in a mobile electronic device having a graphical display and user input device. In certain embodiments, the application further adapts the controller to receive at least one of the possible patient positions via a user input device, and store the received at least one possible patient position in the memory device.

Other embodiments provide for a method for selecting a patient position and an equipment configuration for a medical procedure. The method includes retrieving, via a controller from a memory device, one or more possible patient positions for the medical procedure and one or more possible equipment configurations for the medical procedure. The method further includes selecting, via the controller and based at least in part on one or more parameters of the medical procedure, a patient position from the possible patient positions and an equipment configuration from the possible equipment configurations. The method further includes generating, via the controller, an indicator that conveys the selected patient position and the equipment configuration. In certain embodiments, the one or more parameters of the medical procedure include at least one of: room configuration data; patient physiology data; and medical data. In the presently claimed invention, the medical procedure is a breast biopsy. In certain embodiments, the one or more possible patient positions include at least one of sitting and recumbent. In certain embodiments, the possible equipment configurations include an approach angle for a biopsy probe and/or a needle length of the biopsy probe. In certain embodiments, the method further includes receiving, via a user input device in electronic communication with the controller, medical device data as one of the one or more parameters of the medical procedure. In certain embodiments, the medical device data conveys whether the procedure utilizes a hook or a needle.

Also described is a non-transitory computer readable medium storing instructions. The stored instructions adopt a controller to retrieve, from a memory device, one or more possible patient positions for a medical procedure and one or more possible equipment configurations for the medical procedure. The stored instructions further adapt the controller to select, based at least in part on one or more parameters of the medical procedure, a patient configuration from the possible patient positions and an equipment configuration from the possible equipment configurations, and to generate an indicator that conveys the selected patient position and equipment configuration. In certain examples, one or more parameters of the medical procedure include at least one of room configuration data; patient physiology data; and medical device data. In certain examples, the medical procedure is a breast biopsy.

Accordingly, by selecting a patient position and/or an equipment configuration based on one or more parameters of a medical procedure, some embodiments of the present invention improve the efficacy and/or patient throughput of a medical facility by enabling a technologist/operator/assistant to quickly enter and/or retrieve data concerning the type of equipment/possible patient positions available in a particular room, the preferences of the patient, and/or the preferences of the radiologist/medical professional performing the medical procedure on the patient. In other words, some embodiments of the present invention facilitate improved coordination of the various setup/configuration factors that effect a medical procedure, as compared to traditional approaches/methods which often involve verbal communications between the involved parties, e.g., the patient, medical assistant, and physician performing the medical procedure.

Additionally, while the dimensions and types of materials described herein are intended to define the parameters of the invention, they are by no means limiting and are exemplary embodiments. Many other embodiments will be apparent to those of skill in the art upon reviewing the above description. The scope of the invention should, therefore, be determined with reference to the appended claims. In the **appended claims, the terms "including" and "in which" are used as** the plain-English **equivalents of the respective terms "comprising" and "wherein." Moreover, in the following claims, terms such as "first," "second," "third," "upper," "lower," "bottom," "top," etc. are used merely as labels, and are not intended to impo**se numerical or positional requirements on their objects. Further, the limitations of the following claims are not written in means-plus-function format are not intended to be interpreted as such, unless and until such claim limitations expressly use the **phrase "means for"** followed by a statement of function void of further structure.

This written description uses examples to disclose several embodiments of the invention, including the best mode, and also to enable one of ordinary skill in the art to practice the embodiments of invention, including making and using any devices or systems and performing any incorporated methods. The patentable scope of the invention is defined by the claims, and may include other examples that occur to one of ordinary skill in the art. Such other examples are intended to be within the scope of the claims if they have structural elements that do not differ from the literal language of the claims.

As used herein, an element or step recited in the singular and proceeded **with the word "a" or "an" should be understood as not excluding plural of said** elements or steps, unless such exclusion is explicitly stated. Furthermore, references to **"one embodiment" of the present invention are not intended to be interpreted as** excluding the existence of additional embodiments that also incorporate the recited features. Moreover, unless explicitly stated to the contrary, embodiments **"comprising," "including," or "having" an element or a plurality of elements having a** particular property may include additional such elements not having that property.

Since certain changes may be made in the above-described invention, without departing from the scope of the invention herein involved, it is intended that all of the subject matter of the above description shown in the accompanying drawings shall be interpreted merely as examples illustrating the inventive concept herein and shall not be construed as limiting the invention.

## Claims

1. A system (10) for selecting a patient position (12, 14, 16) and an equipment configuration (18, 20) for a medical procedure, the system (10) comprising:
a controller (22); and
a memory device (24) operative to store an application (26) that adapts the controller (22) to:
retrieve, from the memory device (24), one or more possible patient positions (28) for the medical procedure and one or more possible equipment configurations (30) for the medical procedure;
select, based at least in part on one or more parameters (32) of the medical procedure, a patient position (12) from the possible patient positions (28) and an equipment configuration (18) from the possible equipment configurations (30); and
generate an indicator (34, 36) that conveys the selected patient position (12) and the equipment configuration (18);
wherein the medical procedure is a breast (136) biopsy.

2. The system (10) of claim 1, wherein the one or more parameters (32) of the medical procedure include at least one of:
room configuration data (128);
patient physiology data (130); and
medical device data (132).

3. The system (10) of claim 1 or claim 2, wherein the one or more possible patient positions (28) include at least one of sitting (12) and recumbent (14).

4. The system (10) of any preceding claim, wherein the possible equipment configurations (30) include an approach angle for a biopsy probe (134) and/or a needle length of the biopsy probe (134).

5. The system (10) of any preceding claim, wherein
the one or more parameters (32) of the medical procedure include medical device data (132), and
the application (26) further adapts the controller (22) to receive the medical device data (132) via a user input device (60, 92).

6. The system (10) of claim 5, wherein the medical device data (132) conveys whether the medical procedure utilizes a hook (134) or a needle (134).

7. The system (10) of claim 2, wherein the patient physiology data (130) includes at least one of:
a lesion location;
a breast (136) density;
a breast (136) thickness; and
an initial distance between a biopsy probe (134) and a breast (136).

8. The system (10) of any preceding claim, wherein the controller (22) is disposed in a mobile electronic device having a graphical display (92) and user input device.

9. The system (10) of any preceding claim, wherein the application (26) further adapts the controller (22) to:
receive at least one of the possible patient positions (28) via a user input device (92); and
store the received at least one possible patient position (28) in the memory device (24).

10. A method for selecting a patient position (12, 14, 16) and an equipment configuration (18, 20) for a medical procedure, the method comprising:
retrieving, via a controller (22) from a memory device (24), one or more possible patient positions (28) for the medical procedure and one or more possible equipment configurations (30) for the medical procedure;
selecting, via the controller (22) and based at least in part on one or more parameters (32) of the medical procedure, a patient position (12) from the possible patient positions (28) and an equipment configuration (18) from the possible equipment configurations (30); and
generating, via the controller (22), an indicator (34, 36) that conveys the selected patient position (12) and the equipment configuration (18);
wherein the medical procedure is a breast (136) biopsy.

11. The method of claim 10, wherein the one or more parameters (32) of the medical procedure include at least one of:
room configuration data (128);
patient physiology data (130); and
medical data (132).

12. The method of claim 10 or claim 11, wherein the one or more possible patient positions (28) include at least one of sitting (12) and recumbent (14).

13. The method of claim 10 or claim 11, wherein the possible equipment configurations (30) include an approach angle for a biopsy probe (134) and/or a needle length of the biopsy probe (134).

## Patentansprüche

1. System (10) zur Auswahl einer Patientenposition (12, 14, 16) und einer Ausrüstungskonfiguration (18, 20) für einen medizinischen Eingriff, wobei das System (10) umfasst:
eine Steuerung (22); und
eine Speichervorrichtung (24), die dazu betreibbar ist, eine Anwendung (26) zu speichern, die die Steuerung (22) anpasst zum:
Abrufen, aus der Speichervorrichtung (24), einer oder mehrerer möglicher Patientenpositionen (28) für den medizinischen Eingriff und einer oder mehrerer möglicher Ausrüstungskonfigurationen (30) für den medizinischen Eingriff;
Auswählen, wenigstens teilweise basierend auf einem oder mehreren Parametern (32) des medizinischen Eingriffs, einer Patientenposition (12) aus den möglichen Patientenpositionen (28) und einer Ausrüstungskonfiguration (18) aus den möglichen Ausrüstungskonfigurationen (30); und
Erzeugen eines Indikators (34, 36), der die ausgewählte Patientenposition (12) und die Ausrüstungskonfiguration (18) vermittelt;
wobei es sich bei dem medizinischen Eingriff um eine Biopsie der Brust (136) handelt.

2. System (10) nach Anspruch 1, wobei die ein oder mehreren Parameter (32) des medizinischen Eingriffs wenigstens einen der folgenden beinhalten:
Daten zur Raumkonfiguration (128);
physiologische Daten des Patienten (130); und
Daten der medizinischen Vorrichtung (132).

3. System (10) nach Anspruch 1 oder Anspruch 2, wobei die ein oder mehreren möglichen Patientenpositionen (28) wenigstens eines von sitzend (12) und liegend (14) beinhalten.

4. System (10) nach einem der vorstehenden Ansprüche, wobei die möglichen Ausrüstungskonfigurationen (30) einen Annäherungswinkel für eine Biopsiesonde (134) und/oder eine Nadellänge der Biopsiesonde (134) beinhalten.

5. System (10) nach einem der vorstehenden Ansprüche, wobei
die ein oder mehreren Parameter (32) des medizinischen Eingriffs Daten der medizinischen Vorrichtung (132) beinhalten und
die Anwendung (26) ferner die Steuerung (22) dazu anpasst, die Daten der medizinischen Vorrichtung (132) über eine Benutzereingabevorrichtung (60, 92) zu empfangen.

6. System (10) nach Anspruch 5, wobei die Daten der medizinischen Vorrichtung (132) vermitteln, ob der medizinische Eingriff unter Verwendung eines Hakens (134) oder einer Nadel (134) erfolgt.

7. System (10) nach Anspruch 2, wobei die physiologischen Daten des Patienten (130) wenigstens eines der folgenden beinhalten:
eine Läsionsstelle;
eine Dichte der Brust (136);
eine Dicke der Brust (136); und
einen Anfangsabstand zwischen einer Biopsiesonde (134) und einer Brust (136).

8. System (10) nach einem der vorstehenden Ansprüche, wobei die Steuerung (22) in einer mobilen elektronischen Vorrichtung mit einer graphischen Anzeige (92) und einer Benutzereingabevorrichtung angeordnet ist.

9. System (10) nach einem der vorstehenden Ansprüche, wobei die Anwendung (26) ferner die Steuerung (22) anpasst zum:
Empfangen wenigstens einer der möglichen Patientenpositionen (28) über eine Benutzereingabevorrichtung (92); und
Speichern der erhaltenen wenigstens einen möglichen Patientenposition (28) in der Speichervorrichtung (24).

10. Verfahren zur Auswahl einer Patientenposition (12, 14, 16) und einer Ausrüstungskonfiguration (18, 20) für einen medizinischen Eingriff, wobei das Verfahren umfasst:
Abrufen, über eine Steuerung (22) aus einer Speichervorrichtung (24), einer oder mehrerer möglicher Patientenpositionen (28) für den medizinischen Eingriff und einer oder mehrerer möglicher Ausrüstungskonfigurationen (30) für den medizinischen Eingriff;
Auswählen, über die Steuerung (22) und wenigstens teilweise basierend auf einem oder mehreren Parametern (32) des medizinischen Eingriffs, einer Patientenposition (12) aus den möglichen Patientenpositionen (28) und einer Ausrüstungskonfiguration (18) aus den möglichen Ausrüstungskonfigurationen (30); und
Erzeugen, über die Steuerung (22), eines Indikators (34, 36), der die ausgewählte Patientenposition (12) und die Ausrüstungskonfiguration (18) vermittelt;
wobei es sich bei dem medizinischen Eingriff um eine Biopsie der Brust (136) handelt.

11. Verfahren nach Anspruch 10, wobei die ein oder mehreren Parameter (32) des medizinischen Eingriffs wenigstens eines der folgenden beinhalten:
Daten zur Raumkonfiguration (128);
physiologische Daten des Patienten (130); und
medizinische Daten (132).

12. Verfahren nach Anspruch 10 oder Anspruch 11, wobei die ein oder mehreren möglichen Patientenpositionen (28) wenigstens eines von sitzend (12) und liegend (14) beinhalten.

13. Verfahren nach Anspruch 10 oder Anspruch 11, wobei die möglichen Ausrüstungskonfigurationen (30) einen Annäherungswinkel für eine Biopsiesonde (134) und/oder eine Nadellänge der Biopsiesonde (134) beinhalten.

## Revendications

1. Système (10) pour sélectionner une position de patient (12, 14, 16) et une configuration d'équipement (18, 20) pour une procédure médicale, le système (10) comprenant :
un dispositif de commande (22) ; et
un dispositif de mémoire (24) utilisable pour stocker une application (26) qui adapte le dispositif de commande (22) pour :
récupérer, à partir du dispositif de mémoire (24), une ou plusieurs positions de patient possibles (28) pour la procédure médicale et une ou plusieurs configurations d'équipement possibles (30) pour la procédure médicale ;
sélectionner, sur la base au moins en partie d'un ou plusieurs paramètres (32) de la procédure médicale, une position de patient (12) parmi les positions de patient possibles (28) et une configuration d'équipement (18) parmi les configurations d'équipement possibles (30) ; et
générer un indicateur (34, 36) qui transmet la position de patient sélectionnée (12) et la configuration d'équipement (18) ;
la procédure médicale étant une biopsie du sein (136).

2. Système (10) selon la revendication 1, le ou les paramètres (32) de la procédure médicale comprenant au moins l'un parmi :
des données de configuration de salle (128) ;
des données de physiologie du patient (130) ; et
des données de dispositif médical (132).

3. Système (10) selon la revendication 1 ou la revendication 2, la ou les positions de patient possibles (28) comprenant au moins l'une parmi une position assise (12) et une position allongée (14).

4. Système (10) selon l'une quelconque des revendications précédentes, les configurations d'équipement possibles (30) comprenant un angle d'approche pour une sonde de biopsie (134) et/ou une longueur d'aiguille de la sonde de biopsie (134).

5. Système (10) selon l'une quelconque des revendications précédentes,
le ou les paramètres (32) de la procédure médicale comprenant des données de dispositif médical (132), et l'application (26) adaptant en outre le dispositif de commande (22) pour recevoir les données de dispositif médical (132) par l'intermédiaire d'un dispositif d'entrée utilisateur (60, 92).

6. Système (10) selon la revendication 5, les données de dispositif médical (132) transmettant si la procédure médicale utilise un crochet (134) ou une aiguille (134).

7. Système (10) selon la revendication 2, les données de physiologie du patient (130) comprenant au moins l'un parmi :
un emplacement de lésion ;
une densité du sein (136) ;
une épaisseur du sein (136) ; et
une distance initiale entre une sonde de biopsie (134) et un sein (136).

8. Système (10) selon l'une quelconque des revendications précédentes, le dispositif de commande (22) étant disposé dans un dispositif électronique mobile ayant un affichage graphique (92) et un dispositif d'entrée utilisateur.

9. Système (10) selon l'une quelconque des revendications précédentes, l'application (26) adaptant en outre le dispositif de commande (22) pour :
recevoir au moins l'une des positions de patient possibles (28) par l'intermédiaire d'un dispositif d'entrée utilisateur (92) ; et
stocker l'au moins une position de patient possible reçue (28) dans le dispositif de mémoire (24).

10. Procédé pour sélectionner une position de patient (12, 14, 16) et une configuration d'équipement (18, 20) pour une procédure médicale, le procédé comprenant :
la récupération, par l'intermédiaire d'un dispositif de commande (22) à partir d'un dispositif de mémoire (24), d'une ou plusieurs positions de patient possibles (28) pour la procédure médicale et d'une ou plusieurs configurations d'équipement possibles (30) pour la procédure médicale ;
la sélection, par l'intermédiaire du dispositif de commande (22) et sur la base au moins en partie d'un ou plusieurs paramètres (32) de la procédure médicale, d'une position de patient (12) parmi les positions de patient possibles (28) et d'une configuration d'équipement (18) parmi les configurations d'équipement possibles (30) ; et
la génération, par l'intermédiaire du dispositif de commande (22), d'un indicateur (34, 36) qui transmet la position de patient sélectionnée (12) et la configuration d'équipement (18) ;
la procédure médicale étant une biopsie du sein (136).

11. Procédé selon la revendication 10, le ou les paramètres (32) de la procédure médicale comprenant au moins l'un parmi :
des données de configuration de salle (128) ;
des données de physiologie du patient (130) ; et
des données médicales (132).

12. Procédé selon la revendication 10 ou la revendication 11, la ou les positions de patient possibles (28) comprenant au moins l'une parmi une position assise (12) et une position allongée (14).

13. Procédé selon la revendication 10 ou la revendication 11, les configurations d'équipement possibles (30) comprenant un angle d'approche pour une sonde de biopsie (134) et/ou une longueur d'aiguille de la sonde de biopsie (134).
